# EUROPEAN PATENT APPLICATION

(11) **EP 4 585 695 A1**
(43) Date of publication of application: **16.07.2025**
(21) Application number: 24151783.8
(22) Date of filing: 15.01.2024
(51) Int. Cl.: C12Q 1/6804

(54) **LABEL AND MARKER FOR ANALYSING A BIOLOGICAL SAMPLE**

(71) Applicant: Leica Microsystems CMS GmbH, 35578 Wetzlar (DE)
(72) Inventor: Alsheimer, Soeren, 35578 Wetzlar (DE)
(74) Representative: Schaumburg und Partner Patentanwälte mbB

(57) **Abstract**

In a first aspect a label (100, 200) for analysing a biological sample is provided. The label (100, 200) comprises at least one first labelling oligonucleotide (102, 202) and a labelling moiety (104) attached to the at least one first labelling oligonucleotide (102, 202). The at least one first labelling oligonucleotide (102, 202) comprises a first reaction moiety (116). Further, either the at least one first labelling oligonucleotide (102, 202) comprises a second reaction moiety (118), or the label (100, 200) further comprises an attachment nucleic acid (206) with at least one first attachment sequence to which the at least one first labelling oligonucleotide (102, 202) is at least partially complementary and the attachment nucleic acid (206) comprises a second reaction moiety (118). Further, the first reaction moiety (116) and the second reaction moiety (118) are configured to react with each other. In further aspects, a marker and a reagent kit for analysing a biological sample are provided. Further a method for generating the label is provided.

## Description

### Technical field

The invention relates to a label for analysing a biological sample comprising at least one first labelling oligonucleotide comprising a first reaction moiety. In further aspects, a marker comprising the label and a method for generating the label are provided.

### Background

Labels and markers are frequently used when analysing biological samples, for example in fluorescent microscopy. Markers for fluorescent microscopy generally comprise affinity reagents such as antibodies and labels attached to the affinity reagents in order to enable specifically attaching the labels to a target analyte in a biological sample. This allows the identification and/or localisation of the target analyte in the biological sample. The ubiquitous use of these markers and labels, for example in cyclical staining methods, results in a drive to improve the efficiency of producing these markers and labels, as well as their robustness in use.

A particular issue with labels and markers known in the art is that each part of the label or marker is often generated individually. In order to assemble the label or marker, the individual parts need to be assembled, often using specific chemistries and linkers, resulting in a complex production. These steps are generally associated with losses due to limited efficiency of the reactions and subsequent purification losses.

### Summary

It is an object to provide labels for analysing a biological sample that are easy to assemble and robust as well as a method to generate the labels.

The aforementioned object is achieved by the subject-matter of the independent claims. Advantageous embodiments are defined in the dependent claims and the following description.

In a first aspect a label for analysing a biological sample is provided. The label comprises at least one first labelling oligonucleotide and a labelling moiety attached to the at least one first labelling oligonucleotide. The at least one first labelling oligonucleotide comprises a first reaction moiety. Further, either the at least one first labelling oligonucleotide comprises a second reaction moiety, or the label further comprises an attachment nucleic acid with at least one first attachment sequence to which the at least one first labelling oligonucleotide is at least partially complementary and the attachment nucleic acid comprises a second reaction moiety. Further, the first reaction moiety and the second reaction moiety are configured to react with each other. Providing label elements that comprise reaction moieties enables a particular robust label. Further, the reaction moieties enable an easy assembly of the label from the label elements.

The labelling moieties are preferably optically detectable. For example, the labelling moieties may be fluorophores or polyynes. Each labelling oligonucleotide may have a plurality of labelling moieties attached. These attached labelling moieties may have the same optical properties or differing optical properties. These optical properties may include fluorescent lifetime, excitation wavelength and emission wavelength.

The first and second reaction moieties may in particular be chemical reaction moieties. Thus, the reaction moieties may in particular be configured to react chemically with each other. The reaction moieties may preferably be attached to their respective oligonucleotide or nucleic acid covalently, in particular to the phosphate backbone of the respective oligonucleotide or nucleic acid.

The reaction moieties are configured to react with each other, in particular, in the presence of a catalyst. Such a catalyst may be an element, for example copper, or a physical input such as radiation at a particular wavelength, or a temperature change, in particular a temperature increase. In particular, the reaction moieties are not nucleotides and the catalyst is in particular not an enzyme. The reaction of the first reaction moiety and the second reaction moiety with each other preferably results in a covalent bond. For example, a covalent bond between the first labelling oligonucleotide and the attachment nucleic acid. In this case, labelling oligonucleotides are initially hybridised to the attachment nucleic acid and subsequently covalently attached via the respective reaction moieties.

The first and the second reaction moieties may in particular react based on a click chemistry. Such a chemistry may be, for example, copper-catalyzed azide-alkyne cycloaddition (CuCAAC), metal-free click reactions based on thiolene radical additions, Diels-Alder reactions, or Michael additions as reviewed in for example: Becer CR, Hoogenboom R, Schubert US. 2009, Click chemistry beyond metal-catalyzed cycloaddition. Angew. Chem. Int. Ed. 48, 2-11. Particularly preferred are CuCAAC with 5-Ethynyl-2'deoxyuridine (5-EdU) and Azide-conjugated dyes as well as Dibenzocyclooctyne (DBCO) to Azide conjugated dyes. The latter of which is copperfree.

Preferably, in case the at least one first labelling oligonucleotide comprises the second reaction moiety, the label further comprises a generating nucleic acid with at least one first attachment sequence to which the at least one first labelling oligonucleotide is at least partially complementary. In this case the generating nucleic acid does not comprise the second reaction moiety. The generating nucleic acid enables easy generation of the label and may in particular be used for generating the label.

Preferably, the labelling oligonucleotide, the attachment nucleic acid and the generating nucleic acid are at least one of a naturally occurring nucleic acid, such as D-DNA, or a nucleic acid analogue. Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these altered. The nucleic acid analogue may be an artificial nucleic acid or a xeno nucleic acid. Specific examples of nucleic acid analogues include: Nucleic acids with modified sugars, such as L-DNA comprising L-pentose, or modification of the ribose at the 2'position with for example 2'-amino, 2'-O-methyl, 2'-fluoro modifications. Further, nucleic acids with modified phosphate backbone such as phosporothioate (PT) or boranophosphate (BP) modifications.

In a preferred embodiment, the second reaction moiety is arranged on the attachment nucleic acid such that the second reaction moiety is in close proximity to the first reaction moiety when the first labelling oligonucleotide is hybridised to the first attachment sequence. This enables efficiently reacting the first and the second reaction moieties with each other. In particular, in close proximity means that the first and second reaction moieties are at a distance such that they may react with each other, if the labelling oligo is hybridised to the first attachment sequence.

Preferably, the first reaction moiety and/or the second reaction moiety may be connected to their respective oligonucleotides via flexible linkers of variable length like for example PEG-linkers. This enables adjusting or varying the position at which the first reaction moiety and/or the second reaction moiety are attached to their respective oligonucleotides whilst ensuring the reaction moieties are at a distance such that they may react with each other.

Preferably, the sequence of the first labelling oligonucleotide is specific, in particular, unique, to the at least one labelling moiety attached to the first labelling oligonucleotide. This enables efficient and targeted assembly of the label from a mixtures of different labelling oligonucleotides with different labelling moieties. Consequently, the first attachment sequence may be specific, in particular unique. In case the label has a plurality of labelling oligonucleotides, each labelling oligonucleotide may be attached at a predetermined position, or unique attachment sequence, along the attachment nucleic acid.

Preferably, the attachment nucleic acid and/or the generating nucleic acid is a DNA origami. This enables efficiently generating complex and compact labels, in particular with a plurality of labelling oligonucleotides.

Preferably, the attachment nucleic acid and/or the generating nucleic acid comprises a plurality of oligonucleotides, each oligonucleotide comprising a third reaction moiety at a first end of the oligonucleotide, and a fourth reaction moiety at a second end of the oligonucleotide. This enables efficiently generating the attachment nucleic acid and/or the generating nucleic acid. Similarly, this enables a robust attachment nucleic acid and/or generating nucleic acid. In particular, the third and fourth reaction moieties are configured to react with each other to form a covalent bond, preferably, in the presence of a catalyst.

Preferably, the label comprises a barcode oligonucleotide. This enables specifically hybridising the label to further elements, for example, to an affinity reagent comprising a complementary oligonucleotide. Thus, the barcode oligonucleotide is configured to specifically attach to an affinity reagent. This enables versatile use of the label, for example for generating a marker. For example, the barcode oligonucleotide may be part of the attachment nucleic acid or partially hybridise to the attachment nucleic acid or generating nucleic acid. The barcode oligonucleotide may further comprise a reaction moiety configured to react with a respective reaction moiety of the attachment nucleic acid or of the first labelling oligonucleotide in order to attach the barcode oligonucleotide covalently.

Preferably, the first reaction moiety and the second reaction moiety form a covalent bond, in particular upon reacting with each other. This enables a robust label based on covalent bonds between elements of the label. A label generated from several elements that only bind to each other via hybridisation is less stable under extreme conditions, consequently such a label also has a shorter shelf-life.

Preferably, the generating nucleic acid and/or the attachment nucleic acid comprises a second attachment sequence. This enables generating a label comprising a plurality of labelling oligonucleotides with labelling moieties. In particular, this enables generating a plurality of labels, each label having labelling moieties with different optical properties. In particular, the first attachment sequence and the second attachment sequence differ from each other. This enables specifically attaching labelling oligonucleotides with predetermined labelling moieties.

In a particularly preferred embodiment, the label comprises a second labelling oligonucleotide at least partially complementary to the second attachment sequence and at least one second labelling moiety attached to the second labelling oligonucleotide. This enables generating labels with a plurality of labelling moieties. The labelling moieties of the first and second labelling oligonucleotide may have the same or different optical properties.

In another aspect, a marker for analysing a biological sample is provided. The marker comprises at least one label as described in the above paragraphs. The marker further comprises an affinity reagent configured to bind specifically to a target analyte of the biological sample. This enables generating robust and easy to assemble markers.

Preferably, the affinity reagent may be an antibody, an antibody fragment, or a nucleic acid based aptamer.

Preferably, the affinity reagent comprises an oligonucleotide at least partially complementary to the barcode oligonucleotide. This enables specifically attaching the label to the affinity reagent.

In a further aspect, a reagent kit for analysing a biological sample is provided. The reagent kit comprises at least one label as described above, and a catalyst configured to catalyse a reaction of the first reaction moiety and the second reaction moiety with each other. This enables generating robust and easy to assemble labels.

Preferably, the reagent kit comprises an affinity reagent configured to bind specifically to a target analyte of the biological sample. This enables generating robust and easy to assemble markers.

In another aspect, a method for generating a label for analysing a biological sample is provided. The method comprises the following steps: providing either at least a first labelling oligonucleotide comprising at least one labelling moiety attached to the first labelling oligonucleotide and a first reaction moiety, and an attachment nucleic acid with at least a first attachment sequence to which the first labelling oligonucleotide is at least partially complementary, the attachment nucleic acid comprising a second reaction moiety, or
at least a first labelling oligonucleotide comprising at least one labelling moiety attached to the first labelling oligonucleotide and further comprising a first reaction moiety and a second reaction moiety, a second labelling oligonucleotide comprising at least one labelling moiety attached to the first labelling oligonucleotide and further comprising a first reaction moiety and a second reaction moiety, and a generating nucleic acid comprising at least a first attachment sequence to which the at least one first labelling oligonucleotide is at least partially complementary and an adjacent second attachment sequence to which the second labelling oligonucleotide is at least partially complementary.

In a further step, the method comprises adding a catalyst in order to catalyse a reaction of the first reaction moiety and the second reaction moiety with each other. This enables generating robust and easy to assemble labels.

The marker, the reagent kit and the method have the same advantages as the label. Further, the marker, the reagent kit and the method may be supplemented with the features of the label described in this document, in particular, the features of the dependent claims of the label.

### Short Description of the Figures

Hereinafter, specific embodiments are described referring to the drawings, wherein:
- Figure 1: is a schematic view of steps to generate a label of a marker, and
- Figure 2: is a schematic view of steps to generate a label of a marker according to a further embodiment.

### Detailed Description

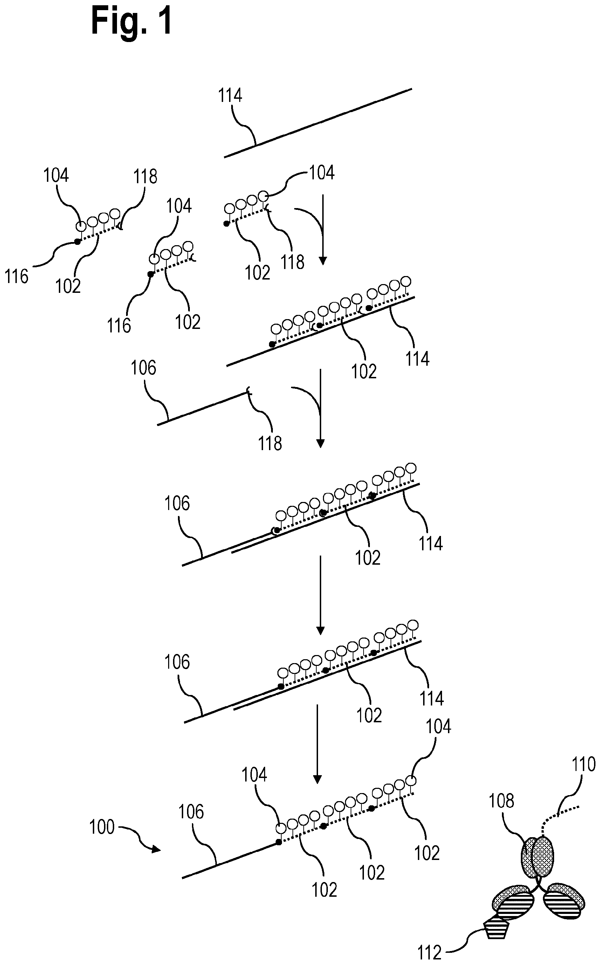

Figure 1 is a is a schematic view of steps to generate a label 100 of a marker. The label 100 comprises three labelling oligonucleotides 102, each labelling oligonucleotide 102 comprises four labelling moieties 104. Further, the label 100 comprises a barcode oligonucleotide 106 for attaching the label 100 to a corresponding affinity reagent 108 comprising an at least partially complementary barcode oligonucleotide 110. The affinity reagent 108 may be an antibody, for example.

The affinity reagent 108 in turn enables binding the marker to a target analyte 112 of a biological sample specifically. Thus, the label 100 with the affinity reagent 108 may be assembled by hybridising the barcode oligonucleotides 106, 110 to each other to give the marker. The marker then enables analysing a biological sample by binding specifically to the target analyte 112 of the biological sample by means of the affinity reagent 108. The label 100 of the marker then enables identifying or locating the target analyte 112 in the biological sample. For example, an optical readout may be generated of the target analyte 112 with the marker and the labelling moieties 104 of the label 100 may be detected, in particular by means of a light microscope.

The label 100 is initially assembled from the three labelling oligonucleotides 102 and a generating nucleic acid 114. The generating nucleic acid 114 may preferably be a continuous stretch of single stranded nucleic acid. The generating nucleic acid 114 comprises three attachment sequences to which the labelling oligonucleotides 102 are at least partially complementary to. The attachment sequences are stretches of the generating nucleic acid 114 that preferably are adjacent to each other along the generating nucleic acid 114. Thus, the labelling oligonucleotides 102 readily hybridise to the generating nucleic acid 114 at the attachment sequences in a first step.

In the case of the embodiment according to Fig. 1, the labelling oligonucleotides 102 all have the same sequence and therefore hybridise equally to the attachment sequences. In an alternative embodiment, the labelling oligonucleotides 102 may have different sequences that correspond to different attachment sequences. This enables hybridising the labelling oligonucleotides 102 at specific attachment sequences or positions along the generating nucleic acid 114.

In a second step, the barcode oligonucleotide 106 is attached to an at least partially complementary sequence of the generating nucleic acid 114 by hybridisation.

The labelling oligonucleotides 102 each comprise a first reaction moiety 116 and a second reaction moiety 118. The reaction moieties 116, 118 are configured to react with each other, in particular, in the presence of a catalyst. Preferably, the first reaction moiety 116 is arranged at a first end of the labelling oligonucleotides 102 and the second reaction moiety 118 is arranged at a second end of the labelling oligonucleotide 102, opposite its first end. Similarly, the barcode oligonucleotide 106 comprises a second reaction moiety 118.

When the barcode oligonucleotide 106 and the three labelling oligonucleotides 102 are attached to the generating nucleic acid 114, the barcode oligonucleotide 106 and the three labelling oligonucleotides 102 are adjacent to each other such that at least the first or the second reaction moiety 116, 118 of one of the barcode oligonucleotide 106 and the three labelling oligonucleotides 102 is adjacent, or in close proximity, to the other one of the first or the second reaction moiety 116, 118 of another of the barcode oligonucleotide 106 and the three labelling oligonucleotides 102. In a third step the first and the second reaction moieties 116, 118 are then caused to react with each other, for example by addition of a catalyst. The reaction may be based on a copper-catalyzed azide-alkyne cycloaddition click chemistry, for example.

As a result, the barcode oligonucleotide 106 and the three labelling oligonucleotides 102 are then linked by the reacted first and second reaction moieties 116, 118. In particular, the barcode oligonucleotide 106 and the three labelling oligonucleotides 102 are covalently linked. This enables a robust link between the barcode oligonucleotide 106 and the three labelling oligonucleotides 102. In an optional further step, the generating nucleic acid 114 may be removed.

The generated label 100 may then be attached to the affinity reagent 108 via the barcode oligonucleotides 106, 110.

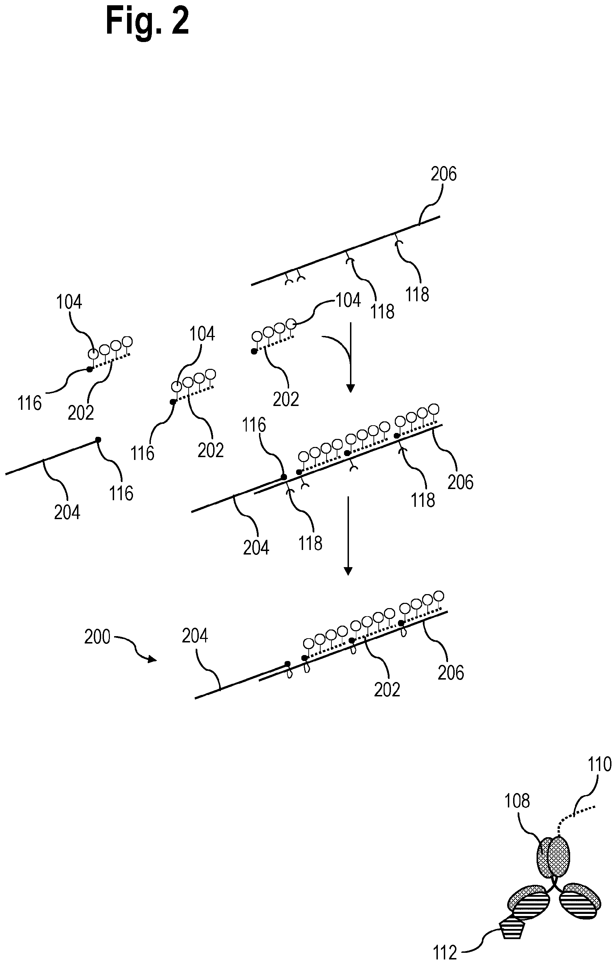

Figure 2 is a is a schematic view of steps to generate a label 200 of a marker. The label 200 comprises three labelling oligonucleotides 202, each labelling oligonucleotide 202 comprises four labelling moieties 104. Further, the label 200 comprises a barcode oligonucleotide 204 for attaching the label 200 to a corresponding affinity reagent 108 comprising the at least partially complementary barcode oligonucleotide 110. The affinity reagent 108 may be an antibody, for example.

The label further comprises an attachment nucleic acid 206, which comprises three attachment sequences to which the labelling oligonucleotides 202 are at least partially complementary to. The attachment sequences are stretches of the attachment nucleic acid 206 that preferably are adjacent to each other along the attachment nucleic acid 206. Thus, the labelling oligonucleotides 202 readily hybridise to the attachment nucleic acid 206 at the attachment sequences in a first step. Further, in the first step the barcode oligonucleotide 204 may attached to a at least partially complementary sequence of the attachment nucleic acid 206 by hybridisation.

In the case of the embodiment according to Fig. 2, the labelling oligonucleotides 202 all have the same sequence and therefore hybridise equally to the attachment sequences. In an alternative embodiment, the labelling oligonucleotides 202 may have different sequences that correspond to different attachment sequences. This enables hybridising the labelling oligonucleotides 202 at specific attachment sequences or positions along the attachment nucleic acid 206.

The labelling oligonucleotides 202 each comprise the first reaction moiety 116. Similarly, the barcode oligonucleotide 204 comprises the first reaction moiety 116. The attachment nucleic acid 206 comprises several of the second reaction moieties 118. In particular, the attachment nucleic acid 206 comprises a number of the second reaction moieties 118 that is equal to the oligonucleotides 202, 204 to be attached to the attachment nucleic acid 206. The second reaction moieties 118 of the attachment nucleic acid 206 are arranged such that they are in close proximity with one of the first reaction moieties 116, when the oligonucleotides 202, 204 are hybridised to the attachment nucleic acid 206, in particular the attachment sequences.

In a second step the first and the second reaction moieties 116, 118 are then caused to react with each other, for example by addition of a catalyst. The reaction may be based on a copper-catalyzed azide-alkyne cycloaddition click chemistry, for example.

As a result, the barcode oligonucleotide 204 and the three labelling oligonucleotides 202 are then linked by the reacted first and second reaction moieties 116, 118 to the attachment nucleic acid 206. In particular, the barcode oligonucleotide 204 and the three labelling oligonucleotides 202 are each covalently linked to the attachment nucleic acid 206. This enables a robust link between the barcode oligonucleotide 204 and the three labelling oligonucleotides 202 via the attachment nucleic acid 206.

The generated label 200 may then be attached to the affinity reagent 108 via the barcode oligonucleotides 204, 110.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

- 100, 200: Label
- 102,202: Labelling oligonucleotide
- 104: Labelling moiety
- 106, 204: Barcode oligonucleotide
- 108: Affinity reagent
- 110: Complementary barcode oligonucleotide
- 112: Target analyte
- 114: Generating nucleic acid
- 116: First reaction moiety
- 118: Second reaction moiety
- 206: Attachment nucleic acid

## Claims

1. A label (100, 200) for analysing a biological sample comprising:
at least one first labelling oligonucleotide (102, 202),
a labelling moiety (104) attached to the at least one first labelling oligonucleotide (102, 202),
wherein the at least one first labelling oligonucleotide (102, 202) comprises a first reaction moiety (116),
wherein either the at least one first labelling oligonucleotide (102, 202) comprises a second reaction moiety (118), or the label (100, 200) further comprises an attachment nucleic acid (206) with at least one first attachment sequence to which the at least one first labelling oligonucleotide (102, 202) is at least partially complementary and the attachment nucleic acid (206) comprises a second reaction moiety (118), and
wherein the first reaction moiety (116) and the second reaction moiety (118) are configured to react with each other.

2. The label according to claim 1, wherein in case the at least one first labelling oligonucleotide (102) comprises the second reaction moiety (118), the label (100) further comprises a generating nucleic acid (114) with at least one first attachment sequence to which the at least one first labelling oligonucleotide (102) is at least partially complementary.

3. The label according to one of the preceding claims, wherein the second reaction moiety (118) is arranged on the attachment nucleic acid (206) such that the second reaction moiety (118) is in close proximity to the first reaction moiety (116) when the first labelling oligonucleotide (202) is hybridised to the first attachment sequence.

4. The label according to one of the preceding claims, wherein the sequence of the first labelling oligonucleotide (102, 202) is specific to the at least one labelling moiety (104) attached to the first labelling oligonucleotide (102, 202).

5. The label according to one of the preceding claims, wherein the attachment nucleic acid (206) and/or the generating nucleic acid (114) is a DNA origami.

6. The label according to one of the preceding claims, wherein the attachment nucleic acid (206) and/or the generating nucleic acid (114) comprises a plurality of oligonucleotides, each oligonucleotide comprising a third reaction moiety at a first end of the oligonucleotide, and a fourth reaction moiety at a second end of the oligonucleotide.

7. The label according to one of the preceding claims, wherein the label comprises a barcode oligonucleotide (106, 204).

8. The label according to one of the preceding claims, wherein the first reaction moiety (116) and the second reaction moiety (118) form a covalent bond.

9. The label according to one of the preceding claims, wherein the generating nucleic acid (114) and/or the attachment nucleic acid (206) comprises a second attachment sequence.

10. The label according to claim 9, comprising a second labelling oligonucleotide (102, 202) at least partially complementary to the second attachment sequence and at least one second labelling moiety (104) attached to the second labelling oligonucleotide (102, 202).

11. A marker for analysing a biological sample comprising:
at least one label (100, 200) according to one of the preceding claims, and
an affinity reagent (108) configured to bind specifically to a target analyte (112) of the biological sample.

12. The marker according to claim 11, wherein the affinity reagent (108) comprises an oligonucleotide (110) at least partially complementary to the barcode oligonucleotide (106, 204).

13. A reagent kit for analysing a biological sample comprising:
at least one label (100, 200) according to one of the claims 1 to 10, and
a catalyst configured to catalyse a reaction of the first reaction moiety (116) and the second reaction moiety (118) with each other.

14. The reagent kit according to claim 13, comprising an affinity reagent (108) configured to bind specifically to a target analyte (112) of the biological sample.

15. A method for generating a label (100, 200) for analysing a biological sample, comprising the following steps:
providing either at least a first labelling oligonucleotide (102, 202) comprising at least one labelling moiety (104) attached to the first labelling oligonucleotide (102, 202) and a first reaction moiety (116), and an attachment nucleic acid (206) with at least a first attachment sequence to which the first labelling oligonucleotide (102, 202) is at least partially complementary, the attachment nucleic acid (206) comprising a second reaction moiety (118), or
at least a first labelling oligonucleotide (102, 202) comprising at least one labelling moiety (104) attached to the first labelling oligonucleotide (102, 202) and further comprising a first reaction moiety (116) and a second reaction moiety (118), a second labelling oligonucleotide (102, 202) comprising at least one labelling moiety (104) attached to the second labelling oligonucleotide and further comprising a first reaction moiety (116) and a second reaction moiety (118), and a generating nucleic acid (114) comprising at least a first attachment sequence to which the at least one first labelling oligonucleotide (102, 202) is at least partially complementary and an adjacent second attachment sequence to which the second labelling oligonucleotide (102, 202) is at least partially complementary,
adding a catalyst in order to catalyse a reaction of the first reaction moiety (116) and the second reaction moiety (118) with each other.
